**Europäisches Patentamt**

**European Patent Office**

**Office européen des brevets**

(19)

(11) **EP 1 313 400 B1**

(12) **EUROPÄISCHE PATENTSCHRIFT**

(45) Veröffentlichungstag und Bekanntmachung des Hinweises auf die Patenterteilung:
**30.11.2005 Patentblatt 2005/48**

(51) Int Cl.⁷: **A61B 17/15**, A61B 5/103

(21) Anmeldenummer: **01984556.9**

(86) Internationale Anmeldenummer:
**PCT/EP2001/005663**

(22) Anmeldetag: **17.05.2001**

(87) Internationale Veröffentlichungsnummer:
**WO 2002/017798 (07.03.2002 Gazette 2002/10)**

(54) **ANORDNUNG ZUR ERMITTLUNG EINER BELASTUNGSACHSE EINER EXTREMITÄT**

DEVICE FOR DETERMINING A LOAD AXIS OF AN EXTREMITY

DISPOSITIF DE DETERMINATION D'UN AXE DE CHARGE D'UNE EXTREMITE

(84) Benannte Vertragsstaaten:
**AT BE CH CY DE DK ES FI FR GB GR IE IT LI LU MC NL PT SE TR**

(30) Priorität: **31.08.2000 DE 10042965**

(43) Veröffentlichungstag der Anmeldung:
**28.05.2003 Patentblatt 2003/22**

(73) Patentinhaber: **PLUS ENDOPROTHETIK AG**
**6343 Rotkreuz (CH)**

(72) Erfinder:
• **BROERS, Holger**
**26670 Uplengen-Spols (DE)**

• **BERNER, Werner**
**CH-5018 Erlinsbach (CH)**
• **HAURI, Bernhard**
**CH-5053 Staffelbach (CH)**
• **HAURI, Thomas**
**CH-5053 Staffelbach (CH)**

(74) Vertreter: **Popp, Eugen et al**
**MEISSNER, BOLTE & PARTNER**
**Postfach 86 06 24**
**81633 München (DE)**

(56) Entgegenhaltungen:
WO-A-00/00093     WO-A-95/00075
WO-A-99/23956     DE-A- 19 709 960
FR-A- 2 785 517

EP 1 313 400 B1

**Beschreibung**

Die Erfindung betrifft eine Anordnung zur Ermittlung der Belastungsachse einer Extremität eines Wirbeltieres gemäß dem Oberbegriff des Anspruches 1.

Belastungsachsen von Extremitäten, wie eines Femurs des Menschen, werden zum korrekten Einsetzen von Prothesen/Implantaten, wie einer Knieprothese, ermittelt. In Abhängigkeit von der Ausrichtung der Belastungsachse des Femurs wird eine für das Einsetzen der Knieprothese am Femur durchzuführende Resektion entsprechend ausgerichtet. Üblicherweise werden die Frontalen des Femurs reseziert. Darüber hinaus erhält der Femur zumindest einen sogenannten Dorsal- sowie einen Ventralschnitt, da der femurseitige Anteil von Knieprothesen üblicherweise U-förmig ausgestaltet ist.

Die exakte Lage der Resektionsflächen am Femur ist für eine lange Lebensdauer der Knieprothese von entscheidender Bedeutung. Bislang ist die Durchführung der Resektion selbst für einen erfahrenen Chirurgen äußerst anspruchsvoll, da während der Operation die Normanlageflächen entsprechend der Vorgabe der Geometrie der Knieprothese unter Berücksichtigung der Belastungsachse festgelegt werden müssen, wobei gegebenenfalls auch pathologische Fehlstellungen zu korrigieren und zudem die Lage und die Wirkung der vorhandenen Bänder und Muskeln zu berücksichtigen sind.

Bekannte Instrumentarien für die Implantation von Knieprothesen umfassen Resektionshilfsmittel in Form von Schneidlehren, welche der Führung eines Sägeblattes dienen. Die Schneidlehren werden hierfür mit Hilfe von unterschiedlichen Vorrichtungen möglichst genau nach der Belastungsachse des Femurs ausgerichtet.

Eine derartige Vorrichtung zur Ausrichtung der Schneidlehre ist aus der WO 00/00093 bekannt. Diese Vorrichtung besteht im wesentlichen aus einem lösbar im distalen Bereich des Femurs arretierbaren Basisteil sowie einem gelenkig und/oder verschiebbar mit dem Basisteil verbundenen Referenzkörper, welcher ein Koordinatensystem bestimmende Mittel aufweist. Die Ausrichtung des Referenzkörpers ist bezüglich dem Femur lagegenau positionierbar, und die das Koordinatensystem bestimmenden Mittel sind zum ausgerichteten Befestigen von Bearbeitungsmitteln, wie einer Schneidlehre, ausgestaltet.

Ein derartiger Referenzkörper ist fest mit dem Femur verbunden und wird in Verlaufsrichtung der Belastungsachse des Femurs ausgerichtet. Ein Ausrichtstab wird zur Ermittlung der Belas-tungsachse verwendet. Dieser Ausrichtstab ist mit dem Referenzkörper an seinem ersten Ende verbunden und wird mit seinem zweiten Ende auf den in der Hüfte liegenden Femurkopf gerichtet, durch den die Belastungsachse des Femurs hindurchläuft. Die Ausrichtung des Ausrichtstabs auf den Femurkopf wird nach Ertasten des Beckenkammes mittels der sogenannten "Zwei-Finger-Methode" durchgeführt. Nachteil der "Zwei-Finger-Methode"-Ertastung ist die relativ hohe Ungenauigkeit.

Aus der US 5,871,018 und der US 5, 682, 886 sind Verfahren zur Ermittlung der Belastungsachse des Femurs bekannt. Gemäß diesen Verfahren werden in einem ersten Schritt die Koordinaten des Femurs beispielsweise durch eine Computertomographieaufnahme ermittelt und in einem Computer abgespeichert. Mit Hilfe der abgespeicherten Daten wird dann ein dreidimensionales Computermodell des Femurs erstellt, und anhand dieses Modells werden die optimalen Koordinaten für das Ansetzen einer Lehre an den Knochen sowie einer anschließend einzusetzenden Knieprothese berechnet. Grundlage hierfür ist die Berechnung der Belastungsachse des Femurs.

Nach einer derartigen Simulation wird der Femur des Patienten fixiert, und mit einer Registrierungseinrichtung werden einzelne Punkte an der Femuroberfläche abgetastet, um die Orientierung des Femurs für die durchzuführende Operation festzustellen. Dieses Abtasten des Knochens erfordert, daß entweder große Teile längs des Femurs möglichst bis zum Hüftgelenk hin offengelegt sein müssen, um deren Oberfläche mit der Registrierungseinrichtung abtasten zu können, oder eine Art Nadel zum Durchstechen der Haut bis auf den Knochen als Abtastinstrument benutzt wird. Da jedoch jeder operative Eingriff für den Patienten ein Risiko darstellt und Nadelstiche Blutergüsse und ein zusätzliches Infektionsrisiko in den Knochenpartien verursachen, ist es nicht wünschenswert, einen zusätzlichen operativen Eingriff im Bereich der Hüfte vorzunehmen oder Nadeleinstiche entlang des Femurs durchzuführen, um den Ort des Rotationszentrums festzustellen. Desweiteren ist eine strenge Fixierung des Femurs auf einem Meßtisch der Registrierungseinrichtung notwendig, da ansonsten Verschiebungen der Hüftpfanne während der Anrastprozedur auftreten und die Schnittlehre nach erfolgter Registrierung der Femurkoordinaten falsch angesetzt werden würde.

Die FR 2 785 517 beschreibt ein Verfahren und eine Vorrichtung zum Detektieren des Rotationszentrums des Femurkopfes in der Hüftpfanne. Hierfür wird der Femur mit seinem Femurkopf in der Hüftpfanne bewegt, und die in verschiedenen Stellungen des Femurs aufgenommenen Meßpunktkoordinaten werden abgespeichert. Sobald eine Verschiebung des Rotationszentrums des Femurs auftritt, wird ein entsprechender Gegendruck auf den Femurkopf ausgeübt, der bei der Bestimmung eines Punktes, der in Beziehung mit der Anordnung des Femurs steht, mit berücksichtigt wird.

Aus der DE 197 09 960 A1 schließlich ist eine Anordnung zur Ermittlung einer Belastungsachse einer mit ihrem ersten Ende in einem nicht fixierten Rotationszentrum drehbar gelagerten Extremität gemäß dem Oberbegriff des Patentanspruchs 1 bekannt. Diese Anordnung umfaßt ein optisches Aufnahmesystem mit fest beabstandeten Kameras zur räumlichen Erfassung von Gebersignalen sowie zwei oder mehr Mehrpunktgeber, wobei ein erster Mehrpunktgeber als beweglicher Taster zum Antasten knöcherner Referenzen zur Bestimmung von deren Koordinaten ausgebildet ist und ein zweiter Mehrpunktgeber ein Meßfeld mit mehreren Meßpunkten zur Erfassung mehrerer Meßpunktkoordinaten umfaßt, sowie eine Auswertungseinheit zur Auswertung der durch die Mehrpunktgeber gelieferten und das optische Aufnahmesystem erfaßten Mehrpunktkoordinatenmengen zur Bestimmung der Belastungsachse der Extremität. Der zweite Mehrpunktgeber kann starr nahe einem zweiten Ende der Extremität, insbesondere nahe dem distalen Femurende angebracht werden derart, daß er in mehreren Drehstellungen der Extremität Meßpunktkoordinatenmengen liefert, die auf Kugelflächen um den jeweiligen Ort des Rotationszentrums liegen. Ferner ist eine Ablaufsteuerung zur Steuerung der sequenziellen Registrierung und Abspeicherung der in den einzelnen Drehstellungen enthaltenen Meßpunktkoordinatenmengen und deren anschließender Verarbeitung nach einem vorbestimmten Verarbeitungsablauf vorgesehen. Bei den Vorrichtungen bzw. Verfahren gemäß dem Stand der Technik ist die Bestimmung einer Belastungsachse relativ kompliziert und somit zeitaufwendig. Ferner kommt es oftmals zu Schwierigkeiten, einen auf die Ermittlung einer Belastungsachse folgenden und auf die Belastungsachse abgestimmten Schnitt mit hoher Präzision durchzuführen.

Der Erfindung liegt die Aufgabe zugrunde, eine Anordnung zur Ermittlung einer Belastungsachse anzugeben, die es erlaubt, die zum anatomisch korrekten Einsetzen eines Implantats in Abhängigkeit von der Orientierung der Belastungsachse der Extremität benötigten Informationen schnell und einfach zu gewinnen, wobei gleichzeitig gewährleistet sein soll, daß ein hinsichtlich der ermittelten Belastungsachse durchgeführter Schnitt mit hoher Präzision bezüglich der Belastungsachse durchgeführt werden kann.

Diese Aufgabe wird durch eine Anordnung nach Patentanspruch 1 gelöst.

Ein wesentlicher Punkt der Erfindung liegt also darin, daß die Anordnung zur Ermittlung der Belastungsachse einen kalibrierten Lehrenadapter aufweist, der mit einer Klammervorrichtung einerseits und einer Schnittlehre zur mechanischen Bearbeitung des zweiten Endes der Extremität andererseits drehfest, aber insbesondere translatorisch verschiebbar, verbindbar ist derart, daß die Schnittlehre über den Lehrenadapter zur Belastungsachse ausrichtbar und längs der Ausrichtungs-Achse verschiebbar ist. Ferner weist die Auswertungseinrichtung Mittel zur Ausführung eines Iterationsvorganges, insbesondere unter Anwendung einer Ausgleichungsrechnung nach der Methode der kleinsten Quadrate zur Bestimmung eines Ortes des Rotationszentrums sowie Mittel zur Berechnung der Belastungsachse aus dem Ort des Rotationszentrums und einem aus den knöchernen Referenzen bestimmten zweiten Punkt am zweiten Ende der Extremität auf. Der erste Mehrpunktgeber sowie ggf. weitere, neben dem zweiten Mehrpunktgeber vorhandene Mehrpunktgeber umfassen jeweils ein Meßpunktfeld mit mehreren Meßpunkten zur Erfassung mehrerer Meßpunktkoordinaten.

In einer bevorzugten Ausführungsform einer erfindungsgemäßen Anordnung dient die verstellbare Klemmvorrichtung als Adapter zur Fixierung des zweiten Mehrpunktgebers nahe dem zweiten Ende der Extremität.

Optional dient der zweite, als Taster ausgeführte Meßpunktgeber auch als Geber zur Ermittlung der aktuellen Position der Schnittlehre.

Vorzugsweise umfaßt eine erfindungsgemäße Anordnung einer Ablaufsteuerung und einer Auswertungseinheit zugeordnete Anzeige- und Eingabemittel zur Realisierung einer optischen und/oder akustischen Menüführung für die Ermittlung des Rotationszentrums und der Belastungsachse.

Die Menüführung kann auch Anzeige- und Eingabemittel für die Positionierung der Schnittlehre umfassen.

Optional umfaßt der zweite Mehrpunktgeber eine Mehrzahl von IR-Leuchtdioden als aktive Meßpunktkoordinaten-Sender, die insbesondere an einem in Art eines Dreibeins ausgebildeten Geber-Grundkörper angebracht sind.

Dem Lehrenadapter können ferner Elektromotoren zur translatorischen Verschiebung der Schnittlehre bezüglich der Belastungsachse zugeordnet sein, die insbesondere durch im Rahmen der Menüführung eingegebene Steuersignale angesteuert werden.

Mit einer erfindungsgemäßen Anordnung wird aus in einer Mehrzahl von Drehstellungen der Extremität mittels des an der Extremität befestigten Mehrpunkt-Gebers erfaßten Meßpunktkoordinatenmengen mindestens eine Gruppe ausgewählt, die einem festen Rotationszentrum zuordenbar ist. Die einem festen Rotationszentrum zugeordneten Meßpunktkoordinatenmengen werden genutzt, um die Belastungsachse der Extremität zu berechnen. Aufgrund des Auswählens mindestens einer Gruppe aus den Meßpunktkoordinatenmengen ist eine Zuordnung des festen Rotationszentrums unter Zurückstellung von fehlerhaften Messungen bzw. von Messungen mit großer Abweichung mittels einer iterativen Ausgleichsrechnung möglich. Somit kann z.B. trotz eines möglichen verschiebens der Beckenpartie des Patienten, in welcher das Rotationszentrum angeordnet ist, während der Drehungen des Beines den Meßpunktkoordinaten ein festes Rotationszentrum zugeordnet werden.

Zur Bestimmung der Belastungsachse werden zwei Punkte ermittelt, durch die die Belastungsachse hindurchläuft. Der erste Punkt ist ein am distalen Ende des Femurs mittels Abtastung der Femuroberfläche ermittelter Referenzpunkt, der sich aus der kniegelenknahen Epikondylen- und Whitesidelinie in seiner Lage bestimmen läßt. Dieser Referenzpunkt ist leicht zu ermitteln, da das distale Ende des Femurs aufgrund des Einsetzens der Knieprothese ohnehin offenliegt. Der zweite Punkt wird durch das der Gruppe von Meßpunktkoordinatenmengen zugeordnete Rotationszentrum dargestellt.

Zur Ermittlung dieses Punktes wird für jeweils eine Drehstellung des Femurs von der Meßeinrichtung eine Erfassung der Meßpunktkoordinaten mittels des am distalen Ende des Femurs befestigten Mehrpunkt-Gebers (aktiven oder passiven Senders) durchgeführt. Hierfür gibt z.B. ein aktiver Sender pro Drehstellung Strahlung aus mehreren Strahlungsquellen ab. Die Strahlungsquellen, wie beispielsweise IR-LED's, sind mit einem festen Abstand und Winkelverhältnis zueinander auf dem Grundkörper angeordnet. In einer anderen Anwendung können die Aktiv-Sender auch durch einen oder mehrere Passiv-Sender ersetzt werden

Aus einer vor Beginn der eigentlichen Messung stattfindenden Kalibrierung des Meßpunkt-Gebers gehen Meßpunktkoordinaten hervor, die die Entfernung und die Winkellage der oben genannten Strahlungsquellen zueinander auf dem Mehrpunkt-Geber wiedergeben. Die aus dieser Kalibrierung gewonnenen Meßpunktkoordinaten werden mit den in jeder Drehstellung erfaßten Meßpunktkoordinaten innerhalb eines Koordinatensystemes verglichen. Anschließend werden diejenigen erfaßten Meßpunktkoordinaten, die nicht innerhalb eines vorbestimmten Toleranzbereiches der Kalibrierungs-Meßpunktkoordinaten liegen, aussortiert, um auf diese Weise grobe Meßfehler der Meßeinrichtung auszuschließen, die beispielsweise durch Fremdreflexe entstehen könnten.

Die bei der vorgeschlagenen Meßanordnung eingesetzten Mehrpunkt-Geber haben mindestens drei, bevorzugt vier, aktive oder passive Strahler (Emitter bzw. Reflektorelemente), die von der zugeordneten Meßeinrichtung - speziell zwei fest beabstandenden Kameras an einem Kamerabalken - registriert werden. Die Emitter bzw. Reflektoren sind jeweils an einem Grundkörper in fester räumlicher Beziehung zueinander angebracht. Da der Grundkörper selbst wiederum fest am Ende der Extremität befestigt ist, deren Rotationsmittelpunkt und Belastungsachse bestimmt werden sollen, beschreiben die "Meßpunkte" im Raum Bewegungsbahnen, die eine feste Zuordnung zueinander haben, wenn die zu untersuchende Extremität um ihr Rotationszentrum geschwenkt wird. Der Operateur hält die Extremität (also beispielsweise das Bein des Patienten mit am distalen Ende des Femurs angebrachtem Mehrpunkt-Geber) in mehreren Drehstellungen jeweils kurz an, und die Meßeinrichtung liefert dann ein Bild von der jeweiligen Lage der einzelnen Meßpunkte.

Wäre das Rotationszentrum während dieser Vorgehensweise exakt fixiert, würden alle Meßpunkte auf Kugelflächen um das Rotationszentrum liegen, und dessen Bestimmung wäre weitgehend trivial. Eine solche Fixierung ist aber in praxi nicht oder jedenfalls nicht streng realisierbar, sondern es finden Verschiebungen des Rotationszentrums während der schrittweisen Schwenkbewegung statt. Ein wesentliches Element ist nun die Extraktion von Gruppen von Meßpunktkoordinatenmengen, die jeweils einem gemeinsamen Rotationszentrum zugeordnet werden können. Während bei einem fixierten Rotationszentrum der Einsatz von Mehrpunkt-Gebern nicht zwingend notwendig wäre, erfordert die vorliegende Verfahrensweise den Einsatz derartiger Geber. Die von diesen gelieferten Koordinatenmengen ermöglichen die Auswertung mit Mitteln in der Ausgleichsrechnung mit hoher Robustheit und Genauigkeit. Aus denjenigen Meßpunktkoordinatenmengen, welche einer Gruppe zuordenbar sind, werden in einer Berechnungseinrichtung der Meßeinrichtung mehrere Kugelflächen berechnet, die die Bewegungsbahnen des zweiten Endes der Extremität, an welchen der Geber angeordnet ist, repräsentieren. Mit Hilfe dieser Kugelflächen lassen die sich Ihnen zugehörigen Mittelpunkte auf einfache und schnelle Weise berechnen. Die berechneten Mittelpunkte entsprechen den Rotationszentren des Femurs bzw. dem Rotationszentrum relativ zum aktiv und/oder passiven Sender. Auf diese Weise ist eine schnelle und einfache Zuordnung des für die Ermittlung der Belastungsachse erforderlichen Rotationszentrums des Femurs im Acetabulum des Beckens möglich, ohne daß ein zusätzlicher operativer Eingriff im Bereich der Hüfte des Patienten notwendig ist.

Relativ zu der auf diese Weise mit hoher Genauigkeit berechneten Belastungsachse des Femurs wird eine am distalen Ende des Femurs zubringende Schnittlehre ausgerichtet, um anschließend eine Schneidvorrichtung (Säge) möglichst präzise und anatomisch korrekt relativ zur Belastungsachse zu führen. Hierfür kann die Schnittlehre über ein Verbindungsteil mit dem am distalen Ende des Femurs bereits angebrachten Halter des festen Mehrpunkt-Gebers verbunden sein und/oder mittels eines zusätzlichen Tasters ausgerichtet werden.

In beiden Fällen findet eine Ausrichtung des Mehrpunkt-Gebers und/oder der Schnittlehre in einem mit seinem Ursprung außerhalb des Patienten angeordneten globalen Koordinatensystemes statt. Dieses bestimmt unter anderem die Anordnung eines mit seinem Ursprung in dem Mehrpunkt-Geber angeordneten lokalen Koordinatensystemes relativ zu den dem Mittelpunkt entsprechenden Rotationszentrum. Da die Schnittlehre und die darin geführte Schneidvorrichtung in Abhängigkeit von der zuvor berechneten Belastungsachse genau ausgerichtet werden kann, kann die Knieprothese an die Schnittflächen des Femurs mit hoher Genauigkeit in bezug auf die Belastungsachse eingesetzt werden.

Durch ein derartiges geometrisch korrektes und genaues Einsetzen der Knieprothese wird gewährleistet, daß der Patient eine optimale Beweglichkeit auch nach der Operation hat.

Durch die Auswahl einer Gruppe von Meßpunktkoordinatenmengen derart, daß möglichst viele Meßpunktkoordinatenmengen miteinander kombiniert werden, wobei jede Meßpunktkoordinatenmenge für eine Drehstellung des Femurs steht, und diese Gruppe von Meßpunktkoordinatenmengen sich jeweils auf ein nicht verschobenes Rotationszentrum bezieht, kann eine während der Messung und/oder zwischen der Messung und dem Ausrichten der Schnittlehre stattfindende Verschiebung des Rotationszentrums durch Veränderung der Patientenlage bei der Bestimmung des Rotationszentrums erkannt und ein verfälschender Einfluß auf die Bestimmung der Belastungsachse ausgeschlossen werden.

In einer bevorzugten Ausführung wird der Schritt des Auswählens einer Gruppe aus den in einer Mehrzahl von Drehstellungen erfaßten Meßpunktkoordinatenmengen mehrfach wiederholt. Nach jeder Auswahl wird diejenige Meßpunktkoordinatenmenge, deren Meßpunktkoordinaten die größte Abweichung von den Koordinaten berechneter Meßpunkte aufweist, zurückgestellt. Die hierfür zu berechnende Abweichung sowie verschiedene Vektorparameter zur Berechnung der Koordinaten des als fest anzunehmenden Rotationszentrums in dem lokalen und dem globalen Koordinatensystem werden mittels der iterativen Ausgleichsrechnung nach der Methode der kleinsten Quadrate ermittelt. Hierbei macht man sich den der Ausgleichsrechnung zugrundeliegenden Grundgedanken der Bestimmung von Näherungswerten für die zu messenden Größen aus fehlerbehafteten Meßwerten zu Nutze, um die für die Abweichung relevante Verbesserung der zu messenden Größen (Meßpunktkoordinaten) anzugeben.

Wichtige Unbekannte sind ein lokaler Translationsvektor für eine Translation eines in dem lokalen Koordinatensystem, dessen Ursprung in dem Sender (aktiv/passiv) angeordnet ist, angeordneten Meßpunktes in das globale System sowie ein globaler Translationsvektor und eine Rotationsmatrix. Die beiden letztgenannten Größen dienen dazu, die Translation eines Meßpunktes zum Rotationspunkt im globalen System sowie die Rotationen des lokalen Systems in dem globalen System zu beschreiben. Durch die Anwendung einer iterativen Ausgleichsrechnung auf die Berechnung des Rotationszentrums ist eine weitestgehend fehlerfreie Ermittlung des Rotationszentrums selbst bei einer Bewegung des Patienten während der Messung möglich.

Eine wichtige Randbedingung ist der Einsatz steriler Meßwerkzeuge sowie einer elektronischen/optischen Meßeinrichtung in Kombination mit der Berechnungseinrichtung, die eine schnellere Durchführung des Meßverfahrens aufgrund optischer Datenübertragungswege und elektronischer Datenverarbeitung und somit eine geringere Operationszeit ermöglicht.

Die Belastungsachse wird mit der computergestützten optischen Meßeinrichtung bestimmt. Sie wird durch das Rotationszentrum und den weiteren Referenzpunkt am distalen Ende des Femurs definiert. Anschließend wird die Schnittlehre mit Hilfe der Meßeinrichtung während des chirurgischen Eingriffs so ausgerichtet, daß sich ihre mechanisch definierten Schnittführungen in korrekter räumlicher Position zur Belastungsachse des Patienten befinden. Hierdurch ist es möglich, daß eine Schneidgenauigkeit von weniger als 1° in Bezug zur Belastungsachse an Abweichung erreicht wird.

Nachfolgend wird die Erfindung anhand von Ausführungsbeispielen beschrieben, die anhand der Abbildungen näher erläutert werden. Hierbei zeigen:

Fig. 1    die Belastungsachse des Femurs;

Fig. 2    die Belastungsachse der Tibia;

Fig. 3    eine Darstellung einer Meßanordnung gemäß einer Ausführungsform der Erfindung;

Fig. 4    eine Darstellung der Koordinatensysteme, die als Berechnungsgrundlage für das Rotationszentrum dienen;

Fig. 5    ein Ablaufdiagramm der Ermittlung einer Belastungsachse;

Fig. 6    eine am Femur angebrachte Schnittlehre mit Mehrfach-Gebern;

Fig. 7    eine Schnittlehre einschließlich eine Vorrichtung zur Positionierung der Schnittlehre am distalen Femurende und

Fig. 8    ein weiteres Ablaufdiagramm eines Verfahrens zur Bestimmung einer Belastungsachse.

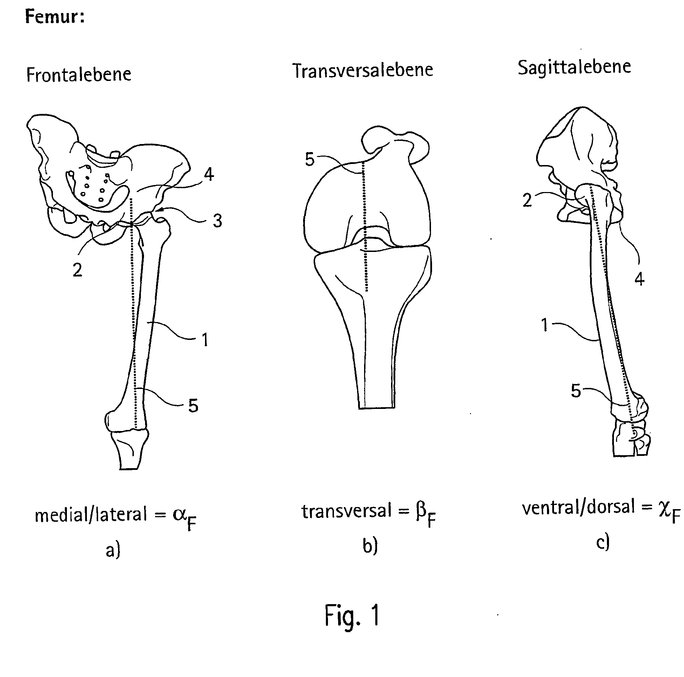

Figur 1 zeigt den Verlauf einer Belastungsachse des Beines am Femur 1 eines gesunden Menschen. Die mit einer gestrichelten Linie eingezeichnete Belastungsachse 5 wird in der unter a) dargestellten Frontalebene in medial/lateraler Richtung $\alpha_F$ verlaufend dargestellt. Die Belastungsachse 5 wird durch ein Rotationszentrum 3 eines Femurkopfes 2 in der Hüftpfanne eines Hüftgelenks 4 und durch knöcherne Referenzen im Bereich des Knies (distalen Femurendes) festgelegt.

In der unter b) dargestellten Transversalebene ist die Richtung $\beta_F$ der Belastungsachse durch den Durchstoßpunkt oder durch die Whitesidelinie und die Richtung der Epikondylenachse bzw. dorsalen Kondylenachse definiert.

In der unter c) dargestellten Sagittalebene ist die ventral/dorsale Richtung $X_F$ der Belastungsachse durch das Rotationszentrum 3 des Femurkopfes 2 und durch knöcherne Referenzen am Femur im Bereich des Knies, ähnlich wie in der Frontalebene, festgelegt.

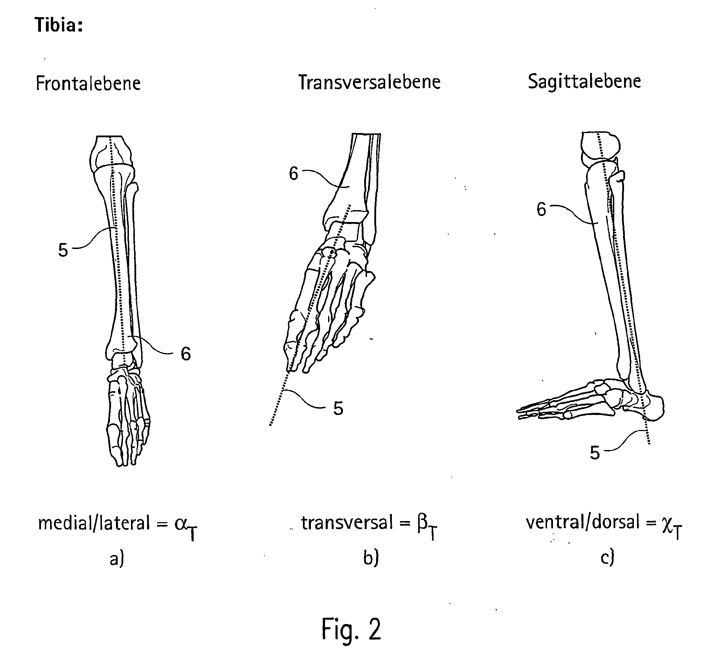

Figur 2 zeigt als weiteres Beispiel für eine Belastungsachse einer Extremität des menschlichen Körpers diejenige Tibia 6. In der unter a) dargestellten Frontalebene ist die medial/laterale Richtung $\alpha_T$ der Belastungsachse 5 durch den Schwerpunkt der Malleolenachse und den Durchstoßpunkt der Belastungsachse 5 einer an entsprechenden knöchernen Referenzen im Knie tibiaseitig definiert. In der unter b) dargestellten Transversalebene ist die Richtung $\beta_T$ der Belastungsachse durch folgende Alternativen festgelegt:

    1. Metatarsaler Strahl,

    2. 23° geneigte Malleolenachse,

    3. Normale der Rotationsachse des oberen Sprunggelenkes oder

    4. aufgrund der Tuberositas.

In der unter c) dargestellten Sagittalebene ist die ventral/dorsale Richtung $X_T$ der Belastungsachse 5 durch die Achse des oberen Sprunggelenks und den Durchstoßpunkt der Belastungsachse 5 des Tibiaplateaus sowie die Kante der vorderen Tibia 6 definiert.

Aus den beiden oben dargestellten Beispielen für eine definierte Belastungsachse wird ersichtlich, daß das Ermitteln der Punkte zum Bestimmen der Ausrichtung bei offen liegenden Knochen und Gelenke relativ einfach durchgeführt werden kann. Wenn jedoch ein zusätzlicher operativer Eingriff, wie er für das Bestimmen des Rotationszentrums 3 im Bereich des Hüftgelenks an sich notwendig wäre, vermieden werden soll, ist eine Ermittlung der Rotationszentrumskoordinaten in jeder der drei oben dargestellten Ebenen allein mit Hilfe von durchgeführten Bewegungen der Extremität wesentlich komplexer.

Es kommt hinzu, daß sich gemäß dem Stand der Technik für eine zuverlässige Ermittlung der Belastungsachse das Rotationszentrum während der für die Ermittlung der Belastungsachse notwendigen Bewegungen des Femurs in seiner Lage nicht verändern sollte, um so einen Neubeginn des Ermittlungsverfahren zu vermeiden. Dies trifft ebenso für den Zeitraum zwischen dem Ermittlungsverfahren und dem Ansetzen der nach der ermittelten Belastungsachse ausgerichteten Schnittlehre am Femur zu.

Wie bereits erwähnt, ist für den Oberschenkel der Verlauf der Belastungsachse 5 durch das Rotationszentrum des Femurkopfes 2 in der Hüftpfanne und einen im Bereich des Knies am distalen Ende des Femurs angeordneten Achsenpunkt definiert. Der am distalen Ende des Femurs befindliche Achsenpunkt wird, da das distale Ende des Femurs ohnehin für das Einsetzen einer Knieprothese offenliegt, aufgrund knöcherner Referenzen festgelegt. Unter knöchernen Referenzen versteht man hierbei spezielle Punkte am distalen Femurende, die in einer bekannten Beziehung zum Verlauf der Belastungsachse des Femurs stehen und die vom Operateur aufgrund von Erfahrungswerten ausgewählt und lokalisiert werden.

Ein solcher Referenzpunkt wird mit Hilfe eines an das distale Ende des Femurs angetasteten Mehrpunkt-Gebers signalisiert. Dieser Mehrpunkt-Geber dient der Erfassung von Meßpunktkoordinaten einer knöchernen Referenz in einer Drehstellung des Femurs. Auch dieser bewegbare Mehrpunkt-Geber (Taster) weist an mehreren Punkten LED's oder Punktreflektoren auf, deren Licht von einem entfernt angeordneten optischen Aufnahmesystem detektiert wird und wodurch den Punkten Koordinaten in einem Koordinatensystem zugeordnet werden.

Da der Femurkopf während der Knieoperation nicht zugänglich ist, muß das Rotationszentrum 3 des Femurs indirekt kinematisch bestimmt werden. Hierfür wird die Stellung des Femurs in verschiedenen Drehstellungen durch die den Ursprung eines globalen Koordinatensystems definierende Meßeinrichtung gemessen.

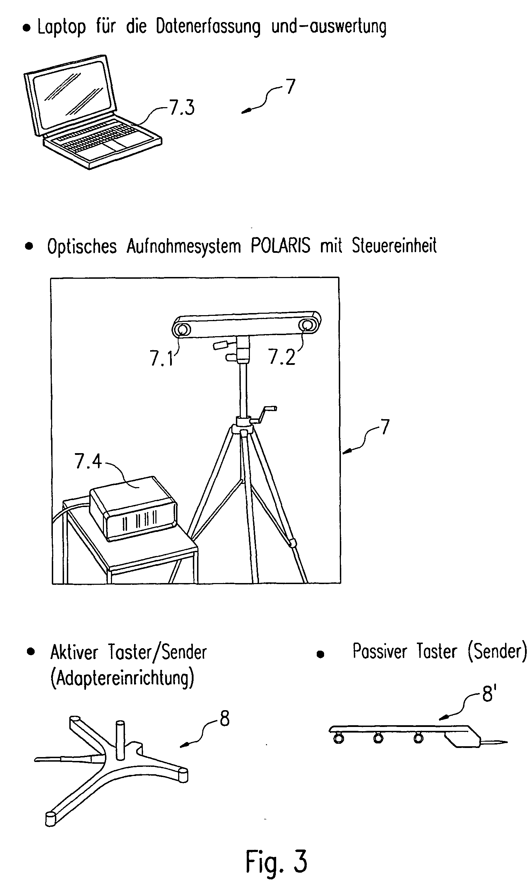

Die wesentlichen Elemente einer solchen Meßeinrichtung sind in Figur 3 dargestellt. Sie umfaßt die bereits oben erwähnten Mehrpunkt-Geber 8 und 8' mit mehreren Leuchtdioden bzw. kleinen Reflektorelementen als koordinatengebende Einheiten, ein optisches Aufnahmesystem 7 mit zwei Kameras 7.1, 7.2 an einem Kamerabalken und eine Steuereinheit 7.4, die dazu dient, die von den LED's emitierende Strahlung zu empfangen, und eine Datenerfassungs- und Auswertungseinheit 7.3, beispielsweise in Form eines Laptops. Erforderlich ist eine Meßeinrichtung mit einem Arbeitsbereich von ca. 0,5 m x 0,5 m x 0,3 m und einer Punktmeßgenauigkeit von ca. 0,5 mm. Eine derartige Meßeinrichtung ist an sich bekannt und wird beispielsweise von Northern Digital Inc. unter der Bezeichnung "Hybrid POLARIS" angeboten. Untersuchungen ergaben eine Meßgenauigkeit von ca. 0,25 mm, womit die Meßeinrichtung die für die Ausrichtung einer Knieprothese gestellten Anforderungen erfüllt.

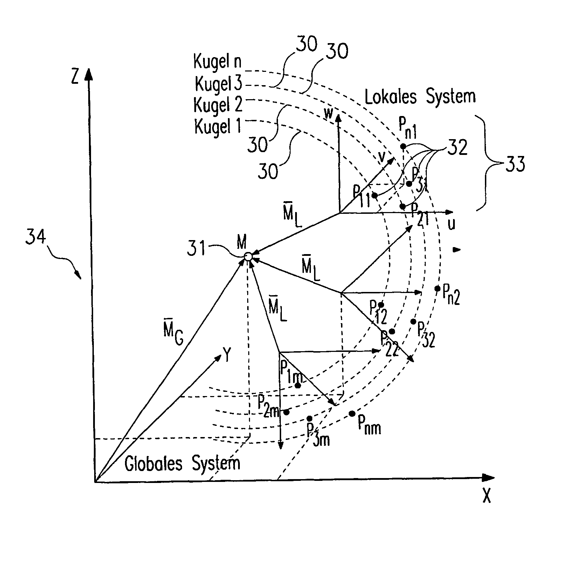

Figur 4 zeigt ein globales und ein lokales Koordinatensystem, die als Berechnungsgrundlage für die Koordinaten des Rotationszentrums dienen. Der mit dem Femur starr verbundene Mehrpunkt-Geber bildet mit den fest zueinander beabstandeten Meßpunkten $P_{11}$-$P_{n1}$, $P_{12}$-$P_{n2}$, $P_{1m}$-$P_{nm}$ als Meßpunktfeld $P_{nm}$ (mit n = Anzahl der Meßpunkte auf dem Mehrpunkt-Geber, m = Anzahl der Femurstellungen) ein lokales Koordinatensystem 33. Der Mehrpunkt-Geber bewegt sich während der Drehbewegungen des Femurs auf den Kugeloberflächen der Kugeln 1, 2, ... n um den gesuchten Mittelpunkt 31 bzw. den Rotationspunkt M, der dem Rotationszentrum 3 in Nähe des Hüftgelenkes entspricht. Der Rotationspunkt M kann durch den globalen Translationsvektor in dem globalen Koordinatensystem 34

$$\vec{G} = (X,\ Y,\ Z),$$

und durch den lokalen Translationsvektor

$$\vec{L} = (u,\ v,\ w)$$

beschrieben werden.

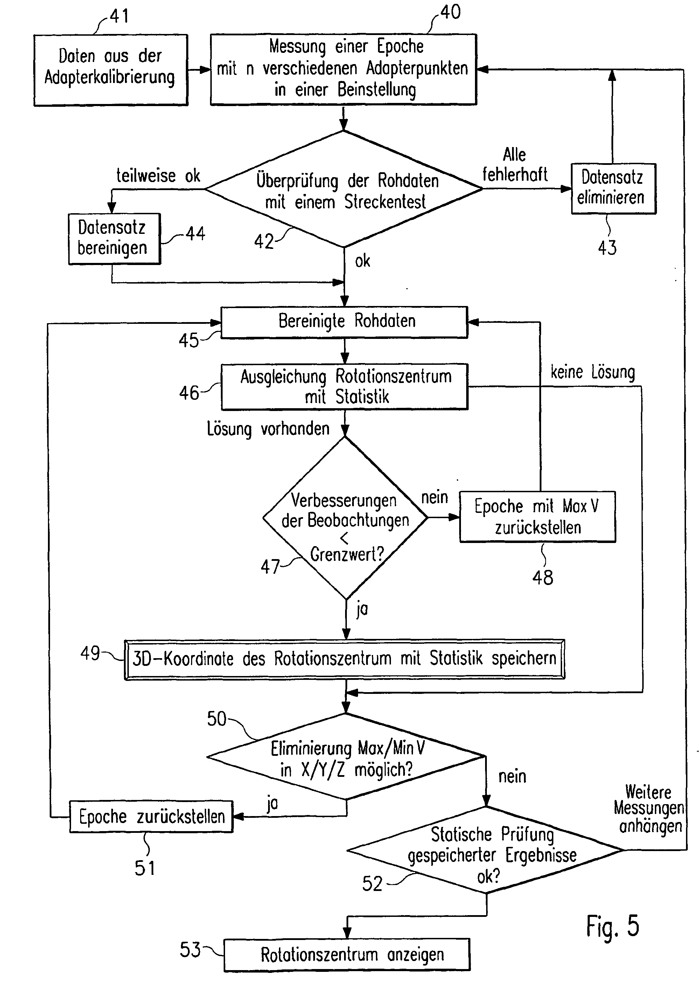

Figur 5 zeigt ein Ablaufdiagramm der Einzelschritte zur Bestimmung des Rotationszentrums. In einem Schritt 40 werden die Meßpunktkoordinaten der Meßpunkte in der Meßeinrichtung für eine bestimmte Drehstellung des Femurs mit Hilfe des Senders (p/a) und des optischen Aufnahmesendesystems erfaßt, um dort mittels einer Datenerfassungs- und Auswerteeinrichtung weiter verarbeitet zu werden.

Zunächst wird in einem Schritt 41 der Sender kalibriert, indem die Abstände und Winkellagen der einzelnen Punkte zueinander auf dem Mehrpunkt-Senders (p/a) in dem lokalen Koordinatensystem festgesetzt werden. Dies erfolgt bei Systemauslieferung. Diese aus der Kalibrierung resultierenden Meßpunktekoordinaten werden sodann in einem Schritt 42 mittels eines sogenannten Streckentests mit den für eine bestimmte Drehstellung erfaßten Meßpunktkoordinaten verglichen. Anschließend werden diejenigen Meßpunktkoordinaten, die außerhalb einer aus der Kalibrierung resultierenden vorbestimmten Toleranz liegen, in einem Schritt 44 aussortiert oder korrigiert, wodurch der Datensatz bereinigt werden kann. Alternativ kann der gesamte Datensatz an Meßpunktkoordinaten bezüglich einer Drehstellung des Femurs (Meßpunktkoordinatenmenge) in einem Schritt 43 eliminiert werden und eine erneute Messung der Meßpunktkoordinaten in einer bestimmten Femurdrehstellung durchgeführt werden. Der Schritt 42 dient somit einer Aussortierung derjenigen Meßpunktkoordinaten, die grobe Meßfehler beinhalten.

Die somit im Schritt 45 aus dem Schritt 44 oder direkt aus dem Schritt 42 erhaltenen bereinigten Meßpunktkoordinaten-Rohdaten werden einer iterativen Ausgleichsrechnung zur Berechnung des Rotationszentrums und damit zur Ermittlung der Belastungsachse unterworfen (Schritt 46).

Die Ausgleichsrechnung dient zur Ermittlung von vorläufigen Ergebnissen für fehlerhafte gemessene Meßpunktkoordinaten und zur Ermittlung von deren Genauigkeit. Die gemessenen Meßpunktkoordinaten werden deshalb als fehlerhaftet angenommen, weil eine Bewegung des Patienten während des Meßvorganges erlaubt sein soll und deshalb eine Verschiebung des Rotationszentrums stattfinden kann.

Die iterative Ausgleichsrechnung findet nach der Methode der kleinsten Quadrate statt. Hierbei wird wie folgt vorgegangen:

Ein Meßpunkt bzw. eine Meßpunktkoordinatenmenge P wird durch die optische Aufnahmevorrichtung gemessen und im globalen Koordinatensystem mit folgendem Ortsvektor beschrieben:

$$\overrightarrow{P_G} = \begin{pmatrix} x_P \\ y_P \\ z_P \end{pmatrix} \qquad (1)$$

Die 3D-Meßpunktkoordinate ist, wie oben bereits erwähnt, mit Bezug auf das lokale Koordinatensystem als Ortsvektor $\vec{P}_L$ bereits vorbestimmt:

$$\overrightarrow{P_L} = \begin{pmatrix} u_P \\ v_P \\ w_P \end{pmatrix} \qquad .$$

$$(2)$$

Die drei unbekannten Parameter, die in die Ausgleichsrechnung miteinfließen, sind die folgenden:

- das durch eine Rotationsmatrix R im dreidimensionalen Raum beschriebene Drehen des lokalen Koordinatensystems in dem globalen Koordinatensystem:

$$R = \begin{pmatrix} a_{11} & a_{12} & a_{13} \\ a_{21} & a_{22} & a_{23} \\ a_{31} & a_{32} & a_{33} \end{pmatrix} \qquad (3)$$

- die Translation des Rotationspunktes M im globalen System, um den die Kugelflächen der Drehbewegungsbahnen des Femur herumverlaufen:

$$\overline{M_G} = \begin{pmatrix} X_M \\ Y_M \\ Z_M \end{pmatrix}$$

$$(4)$$

- die Translation zum Rotationspunkt L im lokalen System:

$$\overline{M_L} = \begin{pmatrix} u_M \\ v_M \\ w_M \end{pmatrix} \qquad (5)$$

Zur Berechnung der Unbekannten und der sich daraus ergebenden Fehlerquadrate bzw. Verbesserungen v wird ein Gleichungssystem aufgestellt, das auf folgender Bedingungsgleichung basiert:

$$\overrightarrow{P_G} = R * \left( \overrightarrow{P_L} - \overrightarrow{M_L} \right) + \overrightarrow{M_G}$$

$$(6)$$

Wenn in der in Vektorschreibweise dargestellten Bedingungsgleichung die Koordinatenparameter gemäß der Gleichungen (1) bis (5) eingesetzt werden, so ergibt sich daraus folgende Gleichung:

$$\begin{pmatrix} X_P \\ Y_P \\ Z_P \end{pmatrix} = \begin{pmatrix} a_{11} & a_{12} & a_{13} \\ a_{21} & a_{22} & a_{23} \\ a_{31} & a_{32} & a_{33} \end{pmatrix} * \begin{pmatrix} u_P - u_M \\ v_P - v_M \\ w_P - w_M \end{pmatrix} + \begin{pmatrix} X_M \\ Y_M \\ Z_M \end{pmatrix}$$

Durch Summation der Beobachtungswerte $X_p$, $Y_p$ und $Z_p$ mit den Verbesserungswerten $v_x$, $v_y$ und $V_z$ ergibt sich folgende Verbesserungsgleichung:

$$\overrightarrow{X_P} = X_P + v_x = a_{11}\left(u_P - u_M\right) + a_{12}\left(v_P - v_M\right) + a_{13}\left(w_P - w_M\right) + X_M + v_x$$

$$\overrightarrow{Y_P} = Y_P + v_y = a_{21}\left(u_P - u_M\right) + a_{22}\left(v_P - v_M\right) + a_{23}\left(w_P - w_M\right) + Y_M + v_y$$

$$\overrightarrow{Z_P} = Z_P + v_z = a_{31}\left(u_P - u_M\right) + a_{32}\left(v_P - v_M\right) + a_{33}\left(w_P - w_M\right) + Z_M + v_z$$

Gemäß der Ausgleichsrechnung nach der Methode der kleinsten Quadrate wird nun die Summe der Verbesserungsquadrate berechnet, wobei sich die Verbesserungswerte aus der Standardabweichung der Unbekannten

$$S_x = S_0 * \sqrt{Q_{ii}}$$

ergibt, worin $Q_{ii}$ die Diagonalelemente einer Designmatrix darstellt und so die Standardabweichung einer Gewichtseinheit

$$S_0 = \sqrt{\frac{v^T\,Pv}{n - u}}$$

ist , wobei P das Gewicht der Beobachtung, n die Anzahl der Beobachtungen und u die Anzahl der Unbekannten ist.

Die Summe der Verbesserungsquadrate wird dann partiell nach den oben genannten Unbekannten abgeleitet, und zur Bestimmung des Minimums der Summe werden diese Ableitungen gleich Null gesetzt. Dadurch ergibt sich ein System von Gleichungen für die Unbekannten, die nach den Unbekannten aufgelöst werden. Mit diesen Lösungen können dann die Schätzwerte für die zu messenden Meßpunktkoordinaten berechnet werden. Die dadurch ermittelten Werte der Unbekannten ergeben die Koordinaten des Rotationszentrums, das durch den Mittelpunkt M des globalen Koordinatensystems der Figur 4 dargestellt wird.

Für jede für eine bestimmte Drehstellung des Femurs ermittelte Meßpunktkoordinatenmenge $P_{11}$-$P_{n1}$, $P_{12}$-$P_{n2}$, $P_{1m}$-$P_{nm}$ wird festgestellt, ob die zu dieser Meßpunktkoordinatenmenge zugehörigen Verbesserungswerte unterhalb eines durch die Meßgenauigkeit der Meßeinrichtung 7 vorgegebenen Grenzwertes liegen (Schritt 47). Wenn dies nicht der Fall ist, muß von einer Verschiebung des Rotationszentrums zwischen der Messung zweier Drehstellungen ausgegangen werden. Diejenige Meßpunktkoordinatenmenge, die maximale Verbesserungswerte aufweist, wird in einem Schritt 48 zurückgestellt, wobei diese Betrachtungen getrennt für eine spezifische Koordinatenrichtung X oder Y oder Z gemacht werden können.

Sofern in dem Schritt 47 festgestellt wird, daß die Verbesserungswerte einer Meßpunktkoordinatenmenge unterhalb des zulässigen Grenzwertes liegen, wird in einem Schritt 49 die 3D-Koordinate des entsprechenden Rotationszentrums in Kombination mit einer Statistik gespeichert.

Wenn in dem oben genannten Schritt 48 eine Meßpunktkoordinatenmenge mit maximalen Verbesserungswerten zurückgestellt wird, so wird, ausgehend von Schritt 45, eine erneute Ausgleichsrechnung unter vorangegangener Auswahl mehrerer Meßpunktkoordinatenmengen durchgeführt. Dieser Schritt der erneuten Ausgleichsrechnung wird so oft wiederholt, bis keine Kombinationen von Meßpunktkoordinatenmengen durch Zusammenfassen in eine Gruppe mehr möglich sind. Dies wird in dem Schritt 50 festgestellt. Sofern in dem Schritt 50 festgestellt wird, daß eine weitere Auswahl an Meßpunktkoordinatenmengen in einer Gruppe möglich ist, wird in dem Schritt 51 eine beliebige Meßpunktkoordinatenmenge zurückgestellt und, beginnend bei Schritt 45, eine erneute Ausgleichsrechnung durchgeführt.

Sofern in Schritt 50 festgestellt wird, daß alle Kombinationen von Meßpunktkoordinatenmengen durchgeführt worden sind, wird in einem Schritt 52 nochmals überprüft, ob die gespeicherten Ergebnisse statistisch gesehen innerhalb vorbestimmter Toleranzbereiche liegen. Anschließend wird das oben dargestellte Verfahren für alle weiteren Koordinatenrichtungen wiederholt durchgeführt, um am Ende in dem Schritt 53 die berechneten Koordinaten eines Rotationszentrums anzuzeigen.

Bei dem Verfahren wirkt sich der robuste Ausgleichungsansatz vorteilhaft aus, der sich aufgrund der Mehrzahl an Meßpunkten mit festem Abstand zueinander auf dem Sender ergibt, wodurch eine feste Beziehung der Beobachtungen in dem Gleichungssystem der Ausgleichsrechnung besteht. Dadurch ist eine Berechnung des Rotationszentrums und eine Ermittlung der Belastungsachse selbst bei bewegter Hüfte möglich.

Sobald das Rotationszentrum auf diese Weise ermittelt worden ist, kann eine gedachte Belastungsachse durch den Punkt des Rotationszentrums und den zuvor ermittelten femurseitigen Referenzpunkt am distalen Femurende gezogen werden.

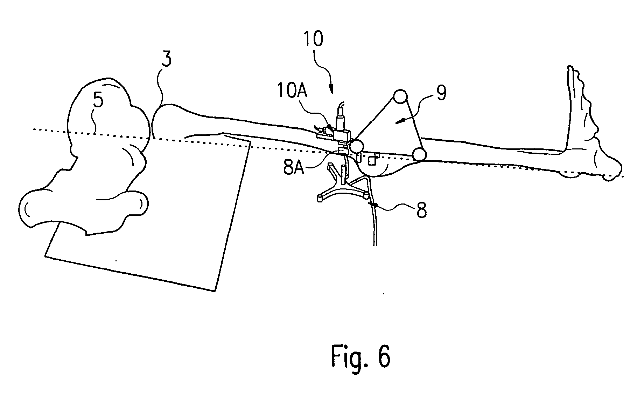

Figur 6 zeigt eine Schnittlehre 10, die bezüglich der ermittelten Belastungsachse 5 des Femurs i' durch einen an einer Haltevorrichtung 8A, die auch den Mehrpunkt-Geber 8 trägt, befestigten Lehrenadapter 10A ausgerichtet ist.

Die Ausrichtung der Schnittlehre 10 bezüglich der ermittelten Belastungsachse 5 des Femurs erfolgt mit Hilfe eines zweiten Gebers 9. Dieser erlaubt eine Ausrichtung der Ebene der Schnittlehre senkrecht zur Belastungsachse. Hierbei wird die Ausrichtung der Belastungsachse 5 dreidimensional betrachtet. Mit dem zweiten Geber 9 (Lehren-Adaptereinrichtung) können Meßpunktkoordinatenergebnisse aus Antastungsvorgängen der Meßeinrichtung 7 im Lehren-Koordinatensystem an die computergestützte Schnittlehre 10 übergeben werden. Diese Ergebnisse können dann für weitere Arbeitsabläufe mit der Schnittlehre 10, wie zum Beispiel ihre Ausrichtung bezüglich der Belastungsachse, weiterverarbeitet werden. Mit der Ausrichtung der Schnittlehre ist dann das Ziel der Vorgabe definierter Schnittebenen erreicht.

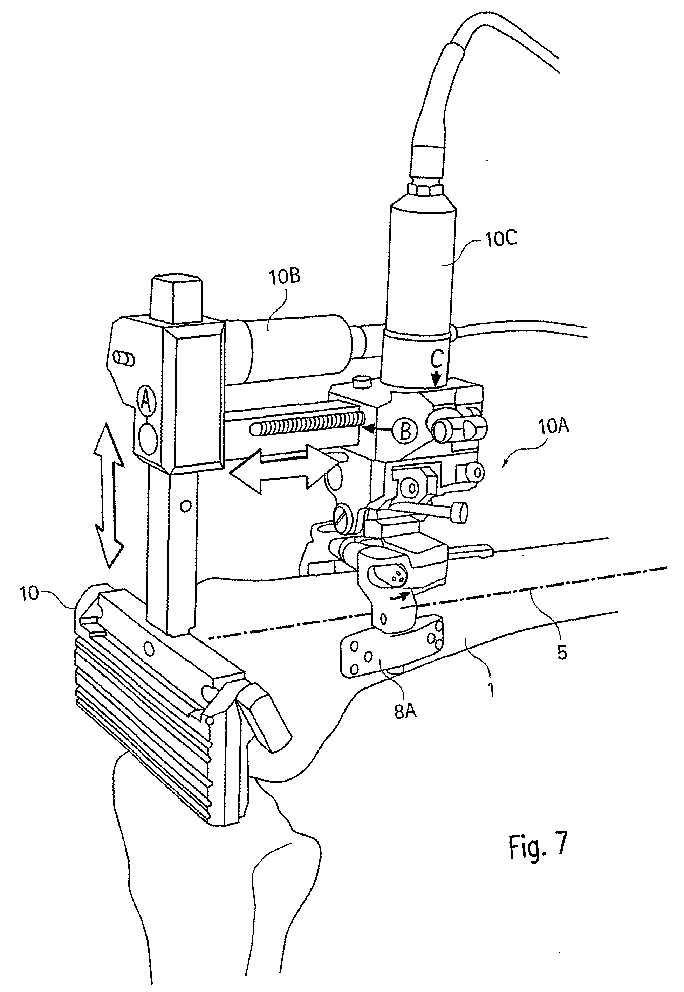

Fig. 7 zeigt den Aufbau einer Anordnung zur Positionierung der Schnittlehre 10 am distalen Ende des Femurs 1 in einer bevorzugten Ausführung noch einmal genauer. Gut zu erkennen ist hier auch die Form der nahe dem distalen Femurende fest angebrachten Klammervorrichtung 8A, die einerseits (bei den Messungen zur Bestimmung der Belastungsachse) den Mehrpunkt-Geber 8 nach Fig. 3 und 6 und andererseits bei den nachfolgenden Resektionsschritten die Schnittlehre 10 trägt.

Auf der Klammervorrichtung 8A ist der kalibrierbare Lehrenadapter 10A angebracht, dessen (nicht einzeln bezeichnete) Winkeleinstellmittel zum einen die Einstellung des Neigungswinkels der Schnittlehrenebene bezüglich der Belastungsachse 5 und zum anderen eine Drehung der Schnittlehre um die Belastungsachse erlauben. Über zwei Elektromotoren 10B und 10C ist - in Verbindung mit (nicht gesondert bezeichneten) Linearantrieben - nach erfolgter Einstellung der Orientierung der Schnittlehre 10 im Raum noch eine Verschiebung in Längsrichtung der Ausrichtungsachse der Schnittlehre 10 sowie senkrecht zu dieser möglich. Hiermit wird die Schnittlehre 10 in Abstimmung auf die für den jeweiligen Patienten als optimal bewertete Prothesengröße in die zugehörigen Resektionsstellungen gebracht. Bei diesen Vorgängen ist wiederum eine exakte Bestimmung der jeweiligen Position der Schnittlehre durch Antasten mit einem beweglichen Mehrpunkt-Geber bzw. Taster 8' (Fig. 3) möglich.

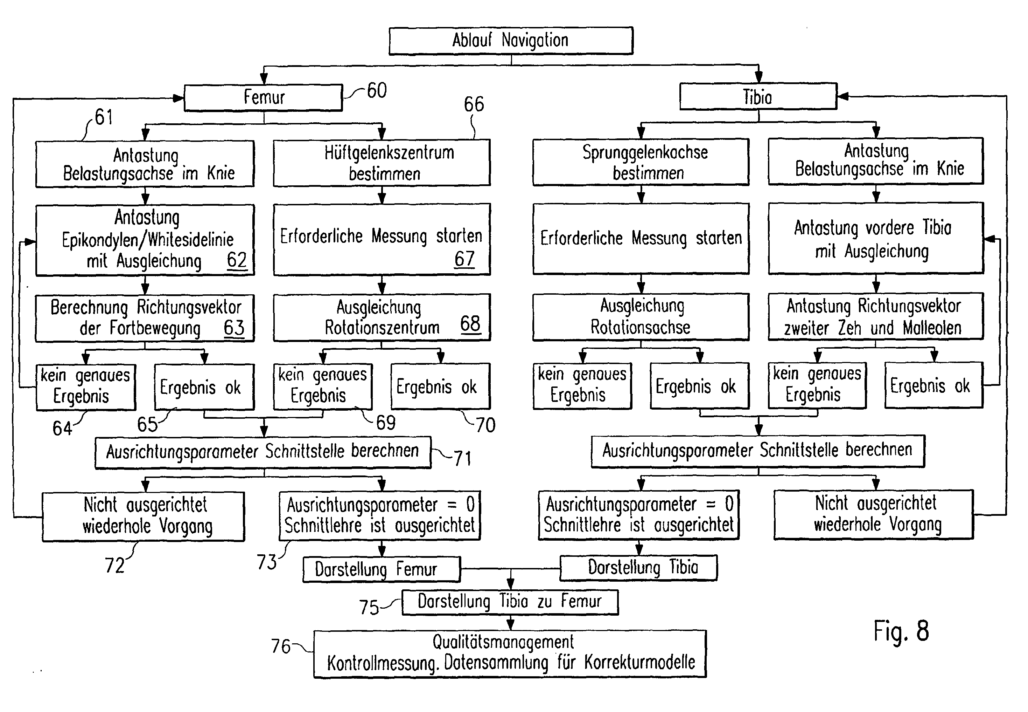

Figur 8 zeigt ein weiteres Ablaufdiagramm, welches schematisch nochmals in groben Zügen die einzelnen Schritte einer bevorzugten Ausführung des Verfahrens zur Bestimmung der Belastungsachse zeigt. Zur näheren Erläuterung wird das Ablaufdiagramm linksseitig bezüglich des Femurs betrachtet.

Zunächst wird in einem Schritt 61 der Referenzpunkt am offenliegenden Ende des Femurs im Bereich des Knies angetastet. Anschließend wird in einem Schritt 62 die Epikondylen- und Widesidelinie angetastet und in einem Schritt 63 der Richtungsvektor der Fortbewegung berechnet.

Sofern sich aus diesen Schritten 61 bis 63 kein genaues Ergebnis ergibt, wird in einem Schritt 64 eine Schleife zurück zu dem Schritt 62 zur Wiederholung dieses Schrittes durchgeführt. Sofern ein genaues Ergebnis in dem Schritt 65 vorliegt, wird dieses zusammen mit dem sich aus dem Schritt 69 ergebenden Ergebnis bezüglich der Berechnung des zweiten Punktes der Belastungsachse, nämlich des Rotationszentrums zur Berechnung der Belastungsachse und der davon abhängigen Ausrichtung der Schnittlehre, in dem Schritt 71 zusammengefaßt.

In dem Schritt 66 soll das Rotationszentrum bestimmt werden, wozu in einem Schritt 67 die erforderlichen Messungen in den einzelnen Drehstellungen des Femurs vorgenommen werden und die erfaßten Meßpunktkoordinaten in einer anschließenden Ausgleichsrechnung im Schritt 68 verarbeitet werden. Auch hier wird, sofern kein genaues Ergebnis in dem Schritt 70 vorliegt, eine Schleife zurück zu dem Schritt 67 gebildet.

Darum wird in dem Schritt 73 die Schnittlehre ausgerichtet ist und wird in einem Schritt 74 der Femur allein. und in einem Schritt 75 der Femur zusammen mit der Tibia für den Operateur mit den gewonnenen Daten dargestellt.

Die oben erläuterten Verfahrensschritte zur Bestimmung der Belastungsachse des Femurs sowie zur Positionierung der Schnittlehre laufen in einer bevorzugten Ausführung des Verfahrens im Rahmen einer interaktiven Menüführung ab, die dem Operateur die einzelnen auszuführenden Schritte und die Zwischenergebnisse der Schritte sowie bestimmte Wahloptionen anzeigt. Die Eingaben seitens des Operateurs erfolgen über eine Tastatur und/oder Fußschalter oder ggf. auch per Sprachsteuerung. In der Menüführung sind bevorzugt klare farbige Grafiken, die auch vom Platz des Chirugen am OP-Tisch gut zu erkennen sind, mit akustischen Signalen (z.B. zur Eingabebestätigung oder als Signal für das Vorliegen brauchbarer Meßergebnisse) kombiniert.

**Bezugszeichenliste**

| | |
|---|---|
| 1 | Femur |
| 2 | Femurkopf |
| 3 | Rotationszentrum |

| | |
|---|---|
| 4 | Hüftgelenk |
| 5 | Belastungsachse |
| 6 | Tibia |
| 7 | Meßeinrichtung |
| 7.1, 7.2 | Kamera |
| 7.3 | Auswerungseinrichtung |
| 7.4 | Steuereinrichtung(Ablaufsteuerung) |
| 8, 8', 9 | Mehrpunkt-Geber |
| 8A | Klammeravorrichtung |
| 10 | Schnittlehre |
| 10A | Lehrenadapter |
| 10B, 10C | Elektromotor |
| 30 | Kugeloberflächenbahn |
| 31 | Mittelpunkt der Kugeln |
| 32 | Meßpunkt |
| 33 | Lokales Koordinatensystem |
| 34 | Globales Koordinatensystem |
| 40 | Messung von Meßpunktkoordinaten |
| 41 | Meßpunktkoordinaten aus der Kalibrierung |
| 42 | Streckentest |
| 43 | Meßpunktkoordinatenmenge eliminieren |
| 44 | Meßpunktkoordinatenmenge bereinigen |
| 45 | Bereinigte Meßpunktkoordinatenmenge |
| 46 | Durchführen der Ausgleichsrechnung mit Statistik |
| 47 | Überprüfung der Grenzwertüberschreitung |
| 48 | Meßpunktkoordinatenmenge mit maximaler Verbesserung zurückstellen |
| 49 | Koordinaten des Rotationszentrums mit Statistik speichern |
| 50 | Überprüfen der Eliminierungsmöglichkeit einer maximalen/minimalen Verbesserung |
| 51 | Meßpunktkoordinatenmenge zurückstellen |
| 52 | Statistische Überprüfung der gespeicherten Koordinaten |
| 53 | Koordinaten des Rotationszentrums anzeigen |
| 60 | Ablauf bezüglich des Femurs |
| 61 | Antastung im Knie |
| 62 | Antastung der Epikondylen/Whitesidelinie mit Ausgleichung |
| 63 | Berechnung eines Richtungsvektors der Fortbewegung |
| 64 | Kein genaues Ergebnis festgestellt |
| 65 | Genaues Ergebnis festgestellt |
| 66 | Bestimmen des Hüftgelenkzentrums |
| 67 | Starten einer erforderlichen Messung |
| 68 | Ausgleichsrechnung zu dem Rotationszentrum durchführen |
| 69 | Genaues Ergebnis festgestellt |
| 70 | Kein genaues Ergebnis festgestellt |
| 71 | Berechnen der Ausrichtungsparameter der Schnittstelle |
| 72 | Wiederholen des Vorganges, wenn Schnittstelle nicht ausgerichtet ist |
| 73 | Feststellen, daß Schnittlehre ausgerichtet ist |
| 74 | Darstellung des Femurs |
| 75 | Darstellung des Femurs und der Tibia |
| 76 | Qualitätsmanagement, Kontrollmessung, Datensammlung für Korrekturmodelle etc. |

**Patentansprüche**

1. Anordnung zur Ermittlung einer Belastungsachse (5) einer mit ihrem ersten Ende in einem nicht fixierten Rotationszentrum, insbesondere einer Hüftpfanne, drehbar gelagerten Extremität (1), speziell eines Femurs, mit

   - einem optischen Aufnahmesystem (7) mit mindestens zwei fest beabstandeten Kameras (7.1, 7.2) zur räumlichen Erfassung von Gebersignalen,
   - mindestens zwei Mehrpunktgebern (8, 8'), wobei ein erster Mehrpunktgeber (8') als beweglicher Taster zum Antasten knöcherner Referenzen zur Bestimmung von deren Koordinaten ausgebildet ist, und ein zweiter Mehrpunktgeber (8) ein Meßpunktfeld mit mehreren Meßpunkten zur Erfassung mehrerer Meßpunktkoordinaten ($P_{11}$-$P_{n-1}$) umfaßt,

- einer Auswertungseinheit (7.3) zur Auswertung der durch die Mehrpunktgeber gelieferten und das optische Aufnahmesystem erfaßten Meßpunktkoordinatenmengen zur Bestimmung der Belastungsachse der Extremität,

wobei

- der zweite Mehrpunktgeber (8) starr nahe einem zweiten Ende der Extremität, insbesondere nahe dem distalen Femurende, angebracht werden kann, derart, daß er in mehreren Drehstellungen der Extremität Meßpunktkoordinatenmengen liefert, die auf Kugelflächen um den jeweiligen Ort des Rotationszentrums liegen,
- eine Ablaufsteuerung (7.4) zur Steuerung der sequentiellen Registrierung und Abspeicherung der in den einzelnen Drehstellungen erhaltenen Meßpunktkoordinatenmengen und deren anschließender Verarbeitung nach einem vorbestimmten Verarbeitungsablauf vorgesehen ist,

**dadurch gekennzeichnet, daß**

- die Auswertungseinrichtung (7.3) Mittel zur Ausführung eines Iterationsvorganges, insbesondere unter Anwendung einer Ausgleichungsrechnung nach der Methode der kleinsten Quadrate, zur Bestimmung eines Ortes des Rotationszentrums sowie Mittel zur Berechnung der Belastungsachse aus dem Ort des Rotationszentrums und einem aus den knöchernen Referenzen bestimmten zweiten Punkt am zweiten Ende der Extremität aufweist,
- der erste Mehrpunktgeber (8') sowie ggf. weitere, neben dem zweiten Mehrpunktgeber vorhandene Mehrpunktgeber jeweils ein Meßpunktfeld mit mehreren Meßpunkten zur Erfassung mehrerer Meßpunktkoordinaten umfaßt bzw. umfassen, und
- die Anordnung einen kalibrierten Lehrenadapter (10 A) aufweist, der mit einer Klammervorrichtung (8A) einerseits und einer Schnittlehre (10) zur mechanischen Bearbeitung des zweiten Endes der Extremität andererseits drehfest, aber insbesondere translatorisch verschiebbar, verbindbar ist, derart, daß die Schnittlehre über den Lehrenadapter zur Belastungsachse (5) ausrichtbar und längs der Ausrichtungs-Achse verschiebbar ist.

2. Anordnung nach Anspruch 1,
   **dadurch gekennzeichnet, daß**
   die verstellbare Klemmvorrichtung (8 A) als Adapter zur Fixierung des zweiten Mehrpunktgebers (8) nahe dem zweiten Ende der Extremität dient.

3. Anordnung nach Anspruch 1 oder 2,
   **dadurch gekennzeichnet, daß**
   der erste, als Taster ausgeführte Mehrpunktgeber (8') auch als Geber zur Ermittlung der aktuellen Position der Schnittlehre (10) dient.

4. Anordnung nach einem der Ansprüche 1 bis 3,
   **gekennzeichnet durch**
   einer Ablaufsteuerung (7.4) und einer Auswertungseinheit (7.3) zugeordnete Anzeige- und Eingabemittel zur Realisierung einer optischen und/oder akustischen Menüführung für die Ermittlung des Rotationszentrums und der Belastungsachse (5).

5. Anordnung nach Anspruch 4,
   **dadurch gekennzeichnet, daß**
   die Menüführung auch Anzeige- und Eingabemittel für die Positionierung der Schnittlehre umfaßt.

6. Anordnung nach einem der vorangehenden Ansprüche,
   **dadurch gekennzeichnet, daß**
   mindestens der zweite Mehrpunktgeber (8) eine Mehrzahl von IR-Leuchtdioden als aktive Meßpunktkoordinaten-Sender umfaßt, die insbesondere an einem in Art eines Dreibeins ausgebildeten Geber-Grundkörper angebracht sind.

**7.** Anordnung nach einem der Ansprüche 1 oder 3 bis 6,
**dadurch gekennzeichnet, daß**
dem Lehrenadapter (10 A) Elektromotoren (10 B, 10 C) zur translatorischen Verschiebung der Schnittlehre (10) bezüglich der Belastungsachse zugeordnet sind, die insbesondere durch im Rahmen einer bzw. der Menüführung eingegebene Steuersignale angesteuert werden.

**Claims**

**1.** Arrangement for determining a load axis (5) of a limb (1), in particular a femur, its first end being rotatably supported in a non-fixed center of rotation, in particular a hip socket, comprising

- an optical recording system (7) having at least two cameras (7.1, 7.2) spaced apart from one another by a fixed distance for spatially measuring sensor signals,
- at least two multi-point indicators (8, 8'), whereby a first multi-point indicator (8') is configured as a movable scanner for the tactile scanning of bony reference points in order to determine their coordinates, and a second multi-point indicator (8) comprises a measurement point field having a plurality of measurement points for determining a plurality of measurement point coordinates ($P_{11}$-$P_{n-1}$),
- an evaluation device (7.3) for evaluating the number of measurement point coordinates furnished by the multi-point indicator and detected by the optical recording system in order to determine the load axis of the limb,

whereby

- said second multi-point indicator (8) can be rigidly attached in proximity to a second end of the limb, in particular near the distal end of the femur, such that it provides a number of measurement point coordinates for multiple rotational positions of the limb at spherical surfaces around the respective locations of the center of rotation,
- a process controller (7.4) is provided for controlling the sequential recording and storing of the number of measurement point coordinates obtained from the individual rotational positions and the subsequent processing of same according to a pre-defined processing sequence,

**characterized in that**

- the evaluation device (7.3) comprises means for performing an iteration process, in particular by using an adjustment calculation based on the least-squares method, in order to determine a location of the center of rotation, as well as means for calculating the load axis from the location of the center of rotation and a second point at the second end of the limb determined from the bony reference points,
- the first multi-point indicator (8') as well as any further multi-point indicators provided in addition to said second multi-point indicator each comprise a measurement point field having a plurality of measurement points for determining a plurality of measurement point coordinates,
- a calibrated jig adapter (10A) is provided which is connectable to a clamping device (8A) at one end and to a cutting jig (10) at the other end so as to be non-rotatable yet in particular allow translational displacement for the mechanical processing of the second end of the limb such that the cutting jig can be aligned with the load axis (5) by means of the jig adapter and can be displaced along the alignment axis.

**2.** Arrangement in accordance with claim 1, **characterized in that**
said adjustable clamping device (8A) serves as an adapter for fixing the second multi-point indicator (8) in the proximity of the second end of the limb.

**3.** Arrangement in accordance with claim 1 or 2, **characterized in that**
the first multi-point indicator (8') configured as a scanner also serves as a sensor for determining the current position of said cutting jig (10).

**4.** Arrangement in accordance with one of claims 1 to 3, **characterized by**
display and input means which are allocated to process controller (7.4) and evaluation unit (7.3) for realizing an optical and/or acoustical menu navigation in the detecting of the center of rotation and the load axis (5).

**5.** Arrangement in accordance with claim 4, **characterized in that**
the menu navigation also comprises display and input means for the positioning of the cutting jig (10).

**6.** Arrangement in accordance with one of the preceding claims, **characterized in that** at least the second multi-point indicator (8) comprises a plurality of IR light-emitting diodes disposed in particular in a sensor base configured in tripod-like form as active transmitters of measurement point coordinates.

**7.** Arrangement in accordance with claim 1 or one of claims 3 -6, **characterized in that** electric motors (10B, 10C) are connected to the jig adapter (10A) for the translational displacement of the cutting jig (10) with respect to the load axis, same being controlled in particular by control signals input within the realm of the menu navigation.

**Revendications**

**1.** Configuration pour déterminer un axe de charge (5) d'une extrémité (1) logée de manière pivotante par sa première extrémité dans un centre de rotation non fixé, en particulier une hanche, spécialement d'un fémur, avec

- un système optique de prise de vue (7) comportant au moins deux caméras écartées fixes (7.1, 7.2) pour l'acquisition spatiale de signaux de capteur,
- au moins deux capteurs multipoints (8, 8'), un premier capteur multipoints (8') étant constitué en tant que palpeur mobile pour palper des références osseuses pour en déterminer les coordonnées, et un deuxième capteur multipoints (8) comportant un champ de points de mesure de plusieurs points de mesure pour déterminer plusieurs coordonnées de point de mesure ($P_{11}$ à $P_{n-1}$),
- une unité d'exploitation (7.3) pour exploiter les quantités de coordonnées de point de mesure fournies par les capteurs multipoints et acquis par le système optique de prise de vue pour déterminer l'axe de charge de l'extrémité,

dans laquelle

- le deuxième capteur multipoints (8) peut être monté de façon rigide à proximité d'une deuxième extrémité de l'extrémité, en particulier à proximité de l'extrémité distale du fémur, de manière telle qu'il fournit en plusieurs positions de rotation les quantités de coordonnées de point de mesure de l'extrémité qui figurent sur des surfaces de sphère autour de l'emplacement correspondant du centre de rotation,
- une commande de déroulement (7.4) pour commander l'enregistrement et le stockage séquentiels des quantités de coordonnées de point de mesure obtenues dans les différentes positions de rotation et leur traitement à la suite après un déroulement prédéterminé de traitement est prévue,

**caractérisée en ce que**

- le dispositif d'exploitation (7.3) présente un moyen pour réaliser un processus d'itération, en particulier en utilisant un calcul de lissage selon la méthode des moindres carrés, pour déterminer un emplacement du centre de rotation ainsi qu'un moyen pour calculer l'axe de charge à partir de l'emplacement du centre de rotation et d'un deuxième point à la deuxième extrémité de l'extrémité déterminé de l'une des références osseuses,
- le premier capteur multipoints (8') ainsi qu'éventuellement d'autres, comprend ou comprennent, outre le capteur multipoints figurant à côté du deuxième capteur multipoints, chaque fois un champ de points de mesure comportant plusieurs points de mesure pour acquérir plusieurs coordonnées de points de mesure, et
- la configuration présente un adaptateur étalonné de guide (10A) qui peut être relié par un dispositif de pince (8A) d'une part et un guide de coupe (10) pour le traitement mécanique de la deuxième extrémité de l'extrémité d'autre part, sans pouvoir tourner mais, en particulier, pouvant être déplacé en translation, de manière telle que le guide de coupe puisse être aligné au moyen de l'adaptateur de guide par rapport à l'axe de charge (5) et qu'il puisse être déplacé le long de l'axe d'alignement.

**2.** Configuration selon la revendication 1,
**caractérisée en ce que**
le dispositif réglable de pince (8A) sert d'adaptateur pour fixer le deuxième capteur multipoints (8) à proximité de la deuxième extrémité de l'extrémité.

**3.** Configuration selon la revendication 1 ou 2,
**caractérisée en ce que**
le premier capteur multipoints (8'), réalisé en tant que palpeur, sert également de capteur pour déterminer la position actuelle du guide de coupe (10).

4. Configuration selon l'une quelconque des revendications 1 à 3,
**caractérisée par**
un moyen d'affichage et de saisie affecté à une commande de déroulement (7.4) et une unité d'exploitation (7.3) pour réaliser un guidage par menu optique et/ou acoustique pour la détermination du centre de rotation et de l'axe de charge (5).

5. Configuration selon la revendication 4,
**caractérisée en ce que**
le guidage par menu comprend également des moyens d'affichage et de saisie pour le positionnement du guide de coupe.

6. Configuration selon l'une quelconque des revendications précédentes,
**caractérisée en ce que**
au moins le deuxième capteur multipoints (8) comprend une pluralité de diodes lumineuses IR en tant qu'émetteur actif de coordonnées de point de mesure, qui sont en particulier montées sur un corps de base de capteurs constitué à la manière d'un tripode.

7. Configuration selon l'une quelconque des revendications 1 ou 3 à 6,
**caractérisée en ce que**
à l'adaptateur de guide (10A) sont affectés des moteurs électriques (10B, 10C) pour le déplacement en translation du guide de coupe (10) par rapport à l'axe de charge, qui sont commandés en particulier par des signaux de commande introduits dans le cadre d'un ou du guidage par menu.

**Femur:**

Frontalebene         Transversalebene        Sagittalebene

medial/lateral = $\alpha_F$     transversal = $\beta_F$     ventral/dorsal = $\chi_F$

a)                 b)                 c)

Fig. 1

**Tibia:**

Frontalebene  Transversalebene  Sagittalebene

medial/lateral = $\alpha_T$  transversal = $\beta_T$  ventral/dorsal = $\chi_T$

a)  b)  c)

Fig. 2

- Laptop für die Datenerfassung und-auswertung

7.3    7

- Optisches Aufnahmesystem POLARIS mit Steuereinheit

7.1    7.2

7.4

7

- Aktiver Taster/Sender (Adaptereinrichtung)

8

- Passiver Taster (Sender)

8'

Fig. 3

Fig. 5

Fig. 6

Fig. 7

EP 1 313 400 B1

Ablauf Navigation

Femur — 60

Tibia

61

Antastung Belastungsachse im Knie

Hüftgelenkszentrum bestimmen

Sprunggelenkachse bestimmen

Antastung Belastungsachse im Knie

66

Antastung Epikondylen/Whitesidelinie mit Ausgleichung 62

Erforderliche Messung starten 67

Erforderliche Messung starten

Antastung vordere Tibia mit Ausgleichung

Berechnung Richtungsvektor der Fortbewegung 63

Ausgleichung Rotationszentrum 68

Ausgleichung Rotationsachse

Antastung Richtungsvektor zweiter Zeh und Malleolen

kein genaues Ergebnis

Ergebnis ok

kein genaues Ergebnis

Ergebnis ok

kein genaues Ergebnis

Ergebnis ok

kein genaues Ergebnis

Ergebnis ok

64

65

69

70

Ausrichtungsparameter Schnittstelle berechnen — 71

Ausrichtungsparameter Schnittstelle berechnen

Nicht ausgerichtet wiederhole Vorgang

Ausrichtungsparameter = 0 Schnittlehre ist ausgerichtet

Ausrichtungsparameter = 0 Schnittlehre ist ausgerichtet

Nicht ausgerichtet wiederhole Vorgang

72

73

Darstellung Femur

Darstellung Tibia

75 — Darstellung Tibia zu Femur

76 — Qualitätsmanagement Kontrollmessung. Datensammlung für Korrekturmodelle

Fig. 8